# EUROPEAN PATENT APPLICATION

(11) **EP 4 053 172 A1**
(43) Date of publication of application: **07.09.2022**
(21) Application number: 20886747.3
(22) Date of filing: 11.11.2020
(51) Int. Cl.: C08F 20/38, C09D 183/10, C12M 3/00

(54) **POLYMER COMPOUND AND COATING COMPOSITION**

(30) Priority: 14.11.2019 JP 2019206567
(71) Applicant: Kyushu University, National University Corporation, Nishi-ku Fukuoka-shi Fukuoka 819-0395 (JP)
(72) Inventor: NISHIMURA, Shinnosuke, Fukuoka-shi, Fukuoka 819-0395 (JP); UEDA, Tomoya, Fukuoka-shi, Fukuoka 819-0395 (JP); TANAKA, Masaru, Fukuoka-shi, Fukuoka 819-0395 (JP)
(74) Representative: Hoffmann Eitle
(86) International application number: PCT/JP2020/042021
(87) International publication number: WO 2021/095753

(57) **Abstract**

A polymer compound in which a polymer chain containing a structure contributing to the inclusion of intermediate water is bound as at least a part of the organic component R group of a silsesquioxane.

## Description

### Technical Field

The present invention relates to a novel polymer compound, a surface treatment composition containing the polymer compound and the like. More particularly, the present invention relates to a polymer compound or the like which can be adsorbed on a glass surface or the like to impart bioaffinity to the surface thereof. This application claims priority based on Japanese Patent Application No. 2019-206567 filed in Japan on November 14, 2019, the contents of which are incorporated herein by reference.

### Background Art

In general, it is known that when a biological component such as blood comes into contact with the surfaces of various materials, the surfaces of the materials are recognized as foreign matters and nonspecific adsorption, denaturation, multi-layer adsorption and the like of proteins in biological tissues occur, resulting in activation of coagulation system, complement system, platelet system and the like. Therefore, it is desirable to impart bioaffinity to the surface of a medical device used in contact with a living body in order to prevent the device from being recognized as a foreign substance and causing a foreign substance reaction with a biological component.

As a means for imparting biocompatibility to the surfaces of various medical devices, attempts have been made to artificially synthesize materials having biocompatibility and apply the artificially synthesized materials to the surfaces of medical devices. As such biocompatible materials, 2-methacryloyloxyethyl phosphorylcholine

(MPC) polymer, polyethylene glycol (PEG), poly (2-methoxyethyl acrylate) (PMEA) and the like are typically known. By applying these biocompatible materials to a region where a biological component such as blood comes into contact with the surface of a medical device, the surface of the medical device is prevented from being recognized as a foreign substance, and as a result, biocompatibility such as inhibition of activation of the coagulation system, the complement system, the platelet system and the like is expressed.

MPC polymer is a kind of betaine which keeps electrical neutrality in biological environment, and has a structure in which phospholipid polar groups covering cell membranes of organisms are bonded as side chains of alkyl chains (main chains). That is, the MPC polymer is a polymer exhibiting biocompatibility by having a structure simulating a substance constituting a living body, and excellent biocompatibility such as suppression of adhesion of platelets can be expressed by applying a polymer obtained by dissolving the MPC polymer in water to the surface of a medical device.

On the other hand, PEG (Polyethylene glycol) is a polymer having a repeating unit of -(C₂H₄-O)- which is a chain ether structure, and is known to have excellent biocompatibility in spite of having a structure that is not similar to substances constituting a living body.

The PMEA has a structure in which a side chain having a main structure of -(C₂H₄-O)-, which is a constitutional unit of the PEG, is bonded to an alkyl chain (main chain), and it is known that the PMEA has biocompatibility.

In addition, polymers having a vinyl ether skeleton as a main chain (Patent Document 1), a polymer having a carbonate bond as a main chain (Patent Document 2), a polymer having a polyethylene structure as a main chain (Patent Document 3), and the like are known as polymers having including a chain ether structure or a cyclic ether structure in a side chain. In addition, Patent Document 4 describes that a polymer having a chain-like ether structure of a plurality of units in a side chain portion exhibits bioaffinity, and Patent Document 5 describes that a polymer having a chain-like ether structure in a side chain portion in which the number of constituent carbon atom is changed exhibits bioaffinity.

It has been clarified that a polymer exhibiting the above-mentioned biocompatibility can contain water molecules in a form commonly referred to as "freezing-bound water" (intermediate water). Intermediate water is characterized by the transfer of latent heat associated with the regularization/disordering of water molecules in the temperature range below zero. It is understood as a water molecule whose properties are intermediate between those of non-freezing water, which is strongly confined to a material surface, and free water, which is hardly confined by a material surface. It has been clarified that such intermediate water is observed not only in the above-mentioned polymers but also in various substances derived from living bodies, and it is considered to play an important role in the expression of biocompatibility.

As described above, with regard to the mechanism for generating water molecules in the form of intermediate water when a polymer having a specific structure is hydrated, it has been clarified that the high molecular mobility exhibited by the polymer having the specific structure is related (see, for example, Non-Patent Document 1). In other words, it is considered that the high molecular mobility of the polymer when it is hydrated is the cause of the formation of water molecules in the form of intermediate water, and as a result, the biocompatibility is exhibited.

### Citation List

### Patent Literature

Patent Literature 1: Japanese Unexamined Patent Publication No. 2014-47347
Patent Literature 2: Japanese Unexamined Patent Publication No. 2014-161675
Patent Literature 3: Japanese Unexamined Patent Publication No. 2014-105221
Patent Literature 4: International Publication WO2004/087228
Patent Literature 5: Japanese Unexamined Patent Publication No. 2017-82174
Patent Literature 6: Japanese Unexamined Patent Publication No. 2016-199620

### Non-Patent Literature

Non-Patent Literature 1: Kagaku, Vol. 66, No. 5, (2011)
Non-Patent Literature 2: Network Polymer, Vol. 32, No. 5, (2011)

### Summary of Invention

### Technical Problem

However, the above-mentioned biocompatible polymers generally have low adhesion to the surface of other materials, and in particular, have poor adhesion to the surface of inorganic materials such as glass and metal, which causes problems when used as a coating agent for covering the surface thereof. Furthermore, many of the biocompatible polymers are difficult to use as coating agents because of their water solubility.

Previous attempts have been made to use biocompatible polymers as coating agents. For example, Patent Document 6 describes a technique for improving coating properties by introducing a side chain having a siloxane bond into a part of the MPC polymer.

Disclosed are of the present invention is to provide a polymer compound having a novel structure that exhibits biocompatibility and good adhesion to various substrates, and a coating composition containing the polymer compound.

### Solution to Problem

In order to solve the above problems, the present invention has the following features.
<1> A polymer compound in which which a polymer chain containing a structure contributing to the inclusion of intermediate water is bonded as at least a part of an organic component R group of a silsesquioxane.
<2> The silsesquioxane is a polymer compound having a structure in which a Si-O-Si bond is broken.
<3> The above-described polymer compound in which the polymer chain containing the structure contributing to the inclusion of the intermediate water is a polymer chain having at least one structure selected from a chain ether structure, a cyclic ether structure and a phospholipid polar group.
<4> The polymer compound according to any one of claims 1 to 3, wherein the ratio of silicon atom contained in the silsesquioxane is 0.5% or more based on the sum of the number of the silicon atom and the number of units of a monomer constituting a polymer chain including a structure contributing to the inclusion of the intermediate water.
<5> The above-described polymer compound in which the mass of intermediate water contained when the polymer compound is saturated with water is 5 mg or more per unit mass of the polymer compound.
<6> A coating composition obtained by dissolving the above-mentioned polymer compound in a solvent.
<7> A medical device wherein at least a part of the surface is coated with the above-mentioned polymer compound.
<8> A support for cell culture, at least a part of the surface of which is coated with the above-mentioned polymer compound.
<9> A method for synthesizing a polymer compound in which a polymer chain containing a structure contributing to the inclusion of intermediate water is bonded to at least a part of organic component R groups of a silsesquioxane, wherein radical polymerization is carried out by mixing a silsesquioxane having a functional group capable of radical vinyl polymerization with a monomer capable of radical vinyl polymerization that contains a structure contributing to the inclusion of intermediate water.

### Advantageous Effects of Invention

The polymer compound according to the present invention is favorably used as a coating composition for imparting biocompatibility to the surfaces of various materials by exhibiting biocompatibility and good adhesion to various substrates.

### BRIEF DESCRIPTION OF THE DRAWINGS

FIG. 1 shows SEC analysis of a random silsesquioxane.
Figure 2A shows a result of NMR analysis (¹H) of a random silsesquioxane.
FIG. 2B shows a result of NMR analysis (¹³C) of a random silsesquioxane.
FIG. 2C shows a result of NMR analysis (²⁹Si) of a random silsesquioxane.
FIG. 3 shows a change in molecular weight (a result of SEC analysis) when PMEA is bound to a random silsesquioxane.
FIG. 4A shows a result of NMR analysis (¹H) of PMEA bound to random silsesquioxane.
FIG. 4B shows a result of NMR analysis (¹³C) of random silsesquioxane with PMEA.
Figure 5 shows a results of DSC analysis of PMEA bonded silsesquioxane.
FIG. 6 shows a glass substrate coated with a polymer compound according to the present invention.
FIG. 7 shows results of a platelet adhesion test on a glass substrate surface coated with a polymer compound according to the present invention.
FIG. 8 shows a change in molecular weight (SEC analysis result) when PMEA is bonded to cage silsesquioxane.
Figure 9A shows a result of NMR analysis (¹H) of PMEA conjugated cage silsesquioxane.
FIG. 9B shows a result of NMR analysis (²⁹Si) of PMEA conjugated cage silsesquioxane.
FIG. 10 shows a glass substrate coated with a polymer compound according to the present invention.

### Description of Embodiments

Since the above-mentioned "intermediate water", which is commonly contained in polymers having bioaffinity, causes the transfer of latent heat associated with the ordering/disordering of water molecules in the temperature range below freezing point (around -30 to -60°C), the amount of intermediate water contained in the sample can be evaluated by measuring the amount of the transfer of latent heat caused by the transformation by DSC or the like.

As a polymer exhibiting biocompatibility by containing intermediate water through hydration, there are known PEG having a structure in which the entire polymer contributes to the inclusion of intermediate water as described above, and polymers exhibiting biocompatibility by adding a side chain containing a phospholipid polar group or a structure contributing to the inclusion of intermediate water such as a chain ether structure or a cyclic ether structure to various polymer main chains. It is considered that a polymer having such biocompatibility is hydrophilic, and when hydrated with water, a part of the hydrated water becomes intermediate water, and as a result, biocompatibility appears. On the other hand, it is considered that the low affinity for a hydrophobic surface such as the surface of an inorganic material such as glass due to the hydrophilic property of the biocompatible polymer causes the low adhesion when the hydrophobic surface is coated.

On the other hand, as a means for improving adhesion when forming a coating or the like containing an organic polymer or the like on the surface of an inorganic base material, there is generally known a means for bringing an organic polymer into close contact with the surface of the organic polymer into close contact with the surface of the inorganic base material by utilizing the characteristic of the Si-O bond.

The present invention is based on the finding that a polymer compound having a structure in which a polymer chain containing a structure contributing to the inclusion of intermediate water is bonded to a compound having a Si-O bond in the form of a silsesquioxane, thereby making it possible to achieve both bioaffinity and adhesion to an inorganic base material, and thus the present invention has been achieved.

Although the mechanism by which the polymer compound according to the present invention achieves both biocompatibility and adhesion to an inorganic base material is not necessarily clear, it is considered from the results of the following examples that the structure having a Si-O bond in the form of a silsesquioxane exhibits adhesion to the inorganic base material while the silsesquioxane structure can contain intermediate water at a predetermined ratio when hydrated for reasons such as not inhibiting the molecular motion of the polymer portion bonded as an organic component thereof.

Silsesquioxane has a silicon atom bonded to one organic constituent (R) and three oxygen atom (R-SiO 1.5). This is a general term for silicon compounds having n as a basic skeleton (Non-Patent Document 2). The silsesquioxane has an arbitrary size ring having a siloxane bond (Si-O) as a minimum unit (the ring may be referred to as an SiO ring hereinafter). SiO rings are linked to each other by sharing a Si-O-Si bond between two rings, and an inorganic compound property is expressed due to the structure constituted by the siloxane bond. On the other hand, since each silicon atom has an organic constituent (R), it is possible to impart an organic compound property caused by the organic constituent.

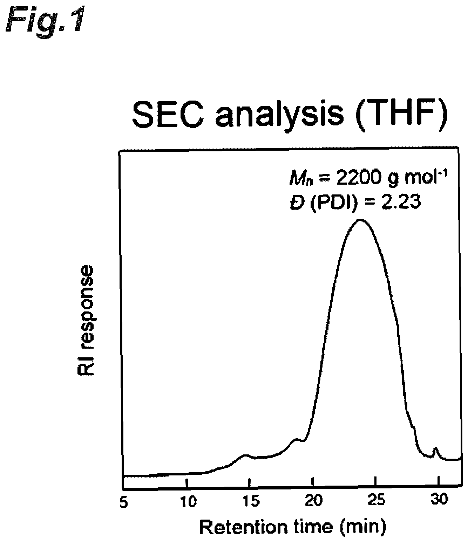

The silsesquioxane has various sizes and shapes depending on the combination of the SiO ring having the Si-O bond as the minimum unit. FIG. 1 shows a typical form of a silsesquioxane. When all the oxygen atom contained in the SiO ring constituting the silsesquioxane are combined with two silicon atom, a cage type (POSS type) silsesquioxane having a closed space surrounded by a plurality of SiO rings as a whole is formed.

On the other hand, there is a silsesquioxane having a structure (Si-O-R') in which a part of the cage silsesquioxane is cleaved and a monovalent functional group or the like (R') is bonded to an oxygen atom bonded to a silicon atom. Examples of the cleaved silsesquioxane having "Si-O-R‴ include a ladder type (Ladder type) in which SiO rings are connected in series and a random type in which SiO rings are connected at random.

The silsesquioxane having the cage type can be distinguished from a cleaved silsesquioxane by, for example, the results of an NMR measurement. That is, since there are few variations in the Si-O bond contained in the cage silsesquioxane, the chemical shift caused by the Si-O bond is relatively sharp, and when the half width of the peak (T3) derived from the silicon atom in which all the silanol groups are condensed is about 50 Hz or less, the content of the cage silsesquioxane is high, and particularly when the half width is about 20 Hz or less, the percentage of the uncondensed silicon atom is sufficiently low. On the other hand, the cleaved silsesquioxane can be distinguished from the cage type silsesquioxane by the existence of variations in the state of the Si-O bond and the broad chemical shift of the peak (T3), particularly at 50 Hz or more. The cleaved silsesquioxane can also be distinguished from the cage silsesquioxane in that a peak originating from the T2 and T1 structures is observed on the low magnetic field side of the peak (T3).

As shown in the following examples, in the case of a polymer compound according to the present invention in which a polymer chain containing a structure contributing to the inclusion of intermediate water in at least a part of the organic component (R) thereof is bonded to a silsesquioxane structure, it was observed that by using a solution obtained by dissolving the polymer compound in an appropriate solvent as a coating agent, adhesion to a glass surface or the like is developed and biocompatibility such as inhibition of platelet adhesion on the surface coated with the polymer compound is developed.

As a result, it is considered that in the polymer compound according to the present invention, the silsesquioxane structure exhibits adhesion to the glass surface and the like, while the properties indicated by the polymer chain containing a structure contributing to the inclusion of intermediate water bonded as the organic component (R) of the silsesquioxane structure are not inhibited by the silsesquioxane structure, the glass surface and the like, and a so-called "polymer brush" state is obtained, and the biocompatibility of the entire surface is exhibited by covering the glass surface as the base material or the portion of the silsesquioxane structure. In this specification, the term "polymer" is used as a term including a polymer which is polymerized to a degree generally referred to as an "oligomer".

In addition, among the polymer compounds according to the present invention, it has been observed that particularly excellent biocompatibility appears on a surface coated using a polymer compound in which a polymer chain containing a structure contributing to the inclusion of intermediate water as an organic component (R) of silsesquioxane having a cage type cleaved by having the above-mentioned "Si-O-R‴ structure is bonded.

This result suggests that in each silicon atom constituting the cage silsesquioxane, as a result of all of the organic components (R) being directed to the outside of the cage, the concentration of the organic components (R) existing between the cage silsesquioxane and the substrate surface becomes high, whereas in the cleaved silsesquioxane, as a result of the cleavage of the Si-O bond, a two dimensional spread is generated, and a portion of the organic components (R) and a portion where three oxygen atoms, centered on the silicon atom, are bonded, are exposed on both sides of the structure, so that when the substrate is brought into contact with the substrate by coating, the adhesion to the substrate is higher than that of the cage silsesquioxane atom.

Based on the above results, a polymer compound in which a polymer chain containing a structure contributing to the inclusion of intermediate water is bonded to at least a part of the organic component R group of the silsesquioxane according to the present invention is specified. According to the polymer compound, a coating composition obtained by dissolving the polymer compound in an appropriate solvent exhibits good adhesion not only to the surface of an organic material but also to a glass surface or the like when applied thereto, and by hydrating the surface on which the coating has been applied, it is possible to express good biocompatibility due to the presence of a structure contributing to the expression of the biocompatibility.

The silsesquioxane contained in the polymer compound according to the present invention can be appropriately produced using known means. For example, a cleaved silsesquioxane containing a structure in which a part of the Si-O-Si bond is cleaved is produced by leaving uncondensed Si-O-R' groups when a monomer having an appropriate organic component (R) bonded to a trialkoxysilane such as trimethoxysilane or triethoxysilane is used to form a silsesquioxane by causing hydrolysis polycondensation or the like to occur between Si-OR' contained in the monomer.

When the molecular weight of the silsesquioxane formed by the above cleavage is small and the degree of condensation of trialkoxysilane or the like is low, the expression of the properties as a silsesquioxane is weakened, and the lowering of adhesion to the substrate surface is observed. Therefore, as the silsesquioxane contained in the polymer compound according to the present invention, a silsesquioxane containing approximately four or more silicon atom and having a siloxane bond between the silicon atom is preferably used. Further, by containing a cleaved silsesquioxane having a larger molecular weight, it is possible to exhibit good adhesion to the surface of the inorganic material.

In the polymer compound according to the present invention, as the organic component (R) of silsesquioxane, a polymer chain containing a structure contributing to the inclusion of intermediate water is bound in an appropriate manner. Examples of the basic structure that contributes to the inclusion of the intermediate water include the above-mentioned phospholipid polar groups, a chain ether structure containing PEG, a cyclic ether structure, and the like, and according to the use of the coating composition using the polymer compound according to the present invention, the polymer compound according to the present invention can be obtained by bonding the polymer chain containing the structure as an organic component (R) of silsesquioxane.

In the polymer compound according to the present invention, the adhesion to glass surfaces and the like is improved by setting the content of silicon atom contained in the silsesquioxane portion (the ratio of silicon atom to the total number of monomer units constituting the polymer bonded to silicon atom as the organic constituent (R)) to about 0.5% or more with respect to the entire polymer compound. When the content of the silicon atom is 1.0% or more, particularly 5.0% or more, the polymer compound according to the present invention can adhere closely to glass surfaces or the like and form surfaces exhibiting good bioaffinity due to the polymer chains containing the structure contributing to the inclusion of the intermediate water. On the other hand, in the case of a polymer compound having a silicon atom content of 10% or more, it is possible to reliably adhere to glass surfaces and the like, while a decrease in the amount of intermediate water that can be contained a decrease in the amount of intermediate water that can be contained.

Examples of polymer chains which can be bonded as the organic component (R) of silsesquioxane by radical vinyl polymerization include those represented by formula (1) below as having a (meth) acrylic skeleton and a chain ether structure. It is known that in the polymer represented by formula (1), a unit constituting PEG is added to the main chain of the (meth) acrylic skeleton by an ester bond, and intermediate water can be contained in many structures.

The polymer represented by formula (1) includes, for example, poly(2-ethoxyethyl acrylate), poly(2-methoxyethyl acrylate), poly[2-(2-methoxyethoxy) ethyl methacrylate], poly[2-(2-ethoxyethoxy)ethyl acrylate], poly [2-(2-methoxyethoxy)ethoxy] ethyl methacrylate], poly[2-(2-methoxyethoxy)ethoxy]ethyl acrylate], poly[2-(2-methoxyethoxy)ethoxy)ethyl acrylate], poly[2-(2-ethoxyethoxy)ethyl methacrylate], poly[2-(2-ethoxyethoxy)ethyl methacrylate], PM2A (poly(2-(2-methoxyethoxy)ethyl acrylate-co-butyl acrylate)), PM3A (poly(2-(2-methoxyethoxy)ethyl acrylate-co-butyl acrylate)), poly (2 - (2-ethoxyethoxyethyl) acrylate), poly (2-ethoxyethylvinylether), poly (tetrahydrofuran-3-ylmethyl acrylate), poly[2-(methoxyethoxy)ethyl methacrylate], and the like. The polymer represented by formula (1) may be used as a polymer by polymerizing only a monomer having a single structure, or may be used as a copolymer containing a plurality of unit structures. 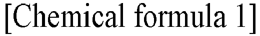 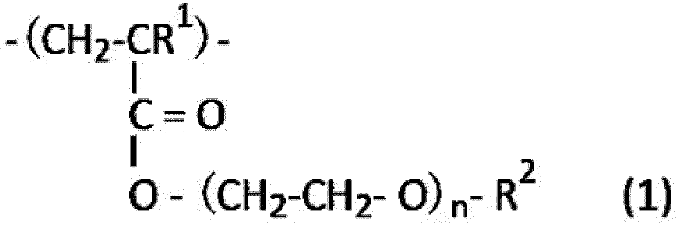 [wherein R¹ is a atom or a methyl group, R² is a methyl or an ethyl group, and n is 1 to 3]

Among the polymers represented by formula (1) above, poly (2-methoxyethyl acrylate (PMEA)) represented by formula (2) below and the like are particularly preferred because they have excellent biocompatibility and are already used for a plurality of bio-related applications. The above-mentioned PMe2A having n=2 as the repeating number (n in formula (1)) of ethylene glycol contained in the side chain of PMEA and the above-mentioned PMe3A having n=3 as the repeating number are also preferably used because they can contain intermediate water at a large ratio.

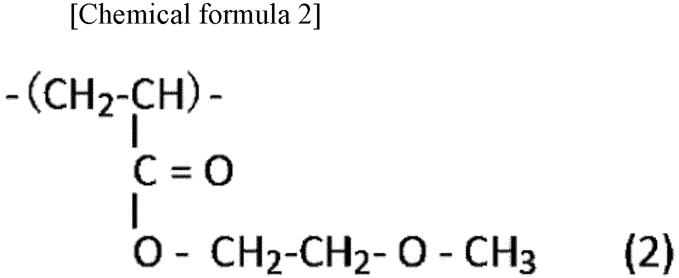

Examples of polymer chains which can be bonded as the organic component (R) of silsesquioxane by radical vinyl polymerization include those represented by the following formulae (3) and (4) as polymer chains having a vinyl skeleton and having a chain ether structure or a cyclic ether structure in the side chain portion. The polymers represented by formulae (3) and (4) have a repeating unit in which a side chain portion containing an ether structure is bonded to the main chain of the vinyl skeleton by an ether bond. The ether structure is a structure of a polyoxyalkylene group which may have a substituent, and as one form thereof, a chain ether which is a chain alkyl oxide represented by general formula (3) or a cyclic ether represented by general formula (4) can be mentioned.

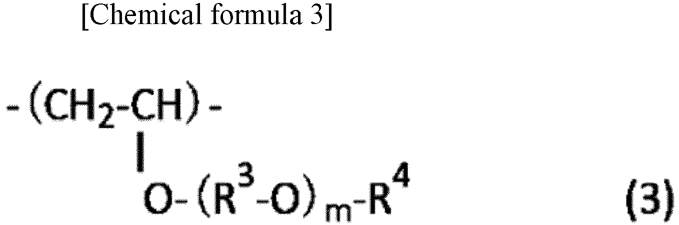

In general formula (3), R³ is a straight chain or branched alkyl group of C1 to C4, and preferably R³ means any one of CH₂, C₂H₄, C₃H₆ and C₄H₈. R⁴ is H or a linear or branched alkyl group of C1 to C4, and preferably R⁴ means any one of H, CH₃, C₂H₅, C₃H₇ or C₄H₉.M is a natural number of 1 to 10, preferably within the range of 1 to 4, and more preferably 1 or 2. Here, the part (R³-O) represents an ether structure which is a unit structure such as PEG.

The polymer compound according to the present invention may contain a repeating unit represented by general formula (3) having mutually different R³, R⁴ and m values. That is, in the polymer compound according to the present invention, the organic component (R) of silsesquioxane may include a structure in which a main chain composed mainly of carbon is bonded to a monoether (m = 1) having a terminal end terminated by a hydrogen or an alkyl group or a polyether (m ≧ 2) by an ether bond so as to form a side chain. For example, when R³ is C₂H₄, the polymer compound of the present invention has a structure in which a chain ether (C₂H₄-O), which is a structural unit of PG terminated with an alkyl group or the like, is bonded to the main chain by an ether bond.

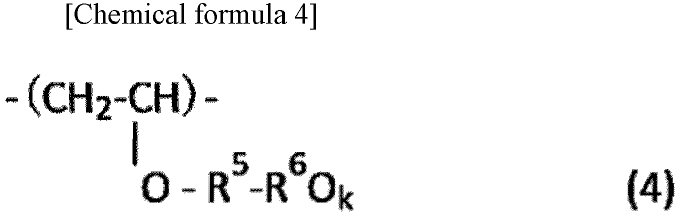

In general formula (4), R⁵ has a structure selected from CH₂ and C₂H₄. R⁶Oₖ is a cyclic ether of any one of a three membered ring and a six membered ring, and the number (k) of the oxygen atom contained in the cyclic ether is k≥1. In the present invention, any hydrogen contained in R⁵ or R⁶ is substituted with at least one of -OH, CH₃ and C₂H₅. That is, the repeating unit of this form has a structure in which a cyclic ether is bonded to the main chain by an ether bond.

In addition to alkoxyalkyl (meth)acrylamide and polyvinyl pyrrolidone (PVP), it is also possible to bond a polymer chain capable of containing intermediate water as an organic component (R) of silsesquioxane by introducing a chain amide structure, a cyclic amide structure or the like into a (meth) acrylic skeleton or the like via an amide group to form a side chain.

For example, when forming a polymer compound in which PMEA represented by formula (2) is bonded to an organic component (R) of silsesquioxane, a desired polymer compound can be obtained by causing radical polymerization to occur in a state in which an MEA monomer is mixed with silsesquioxane in which a structure having radical polymerization property is provided in the organic component (R).

When PEG or the like composed of a structure in which the entire polymer contributes to the inclusion of intermediate water is used, a macromonomer in which a vinyl group or the like is bonded to the terminal of PEG or the like is used and mixed with a silsesquioxane provided with a structure having radical polymerization ability in the organic component (R) an organic component (R) in the same manner as described above, and radical polymerization is carried out, whereby a polymer compound in which a polymer chain in which a plurality of PEGs are bonded in a side chain state is bonded to an organic component (R) of silsesquioxane is obtained.

The degree of biocompatibility indicated by the polymer compound of the present invention is determined according to the amount of intermediate water contained when the polymer compound is hydrated. The amount of the contained intermediate water is determined according to the structure of the polymer bonded to the organic component (R) of the cleaved silsesquioxane, the ratio of the polymer moiety in the polymer compound, and the like. On the other hand, since it is considered that the degree of adhesion of the polymer compound according to the present invention to the substrate is mainly determined by the ratio of the silsesquioxane portion in the polymer compound, it is desirable that a polymer having a structure capable of containing intermediate water at a higher density is bonded to the organic component (R) of silsesquioxane from the viewpoint of achieving both the biocompatibility and adhesion to the substrate in the polymer compound according to the present invention at a higher level.

In the polymer compound according to the present invention, when a polymer having a predetermined structure is used, by increasing the length of the polymer chain bonded to the silsesquioxane or the bonding density thereof, the ratio of the polymer portion in the polymer compound increases, and properties such as biocompatibility possessed by the polymer can be strongly expressed.

On the other hand, with respect to a polymer compound used for a surface to which it is particularly difficult to adhere a coating composition, by lowering the ratio of the polymer portion bonded to the organic component (R) of the silsesquioxane, the properties of the silsesquioxane portion can be strongly expressed and good adhesion can be obtained.

The bond density of the polymer bonded to the organic component (R) of the silsesquioxane can be determined by the density of the radical polymerizable structure present in the organic component (R) of the silsesquioxane used in synthesizing the polymer compound according to the present invention. Typically, a polymer compound having a high polymer bond density can be obtained by introducing an organic constituent (R) having a radical polymerizable structure into all the silicon atom contained in the silsesquioxane.

On the other hand, by introducing an organic constituent (R) having a radical-polymerizable structure only in part of the silicon atom contained in the silsesquioxane, the bonding density of the polymer bonded to the organic constituent (R) of the silsesquioxane is lowered, whereby high adhesion to the substrate can be obtained when used as a coating composition.

The length of the polymer chain bonded as the organic component (R) of silsesquioxane in the polymer compound according to the present invention can be adjusted mainly by the preparation composition at the time of synthesizing the polymer compound. That is, by increasing the ratio of the monomer for constituting the polymer chain bonded to the organic component (R), it is possible to increase the molecular weight of each organic component (R), and it is possible to strongly express properties such as biocompatibility possessed by the polymer.

On the other hand, even if the length of the polymer chain bonded as each organic component (R) is generally an oligomer in which the unit of the monomer is from a quantity to a ten quantity quantity, intermediate water can be contained and properties such as bioaffinity can be expressed.

The degree of biocompatibility of the polymer compound according to the present invention can be evaluated based on the amount of intermediate water that the polymer compound can contain. When the amount of intermediate water that can be contained per unit mass of the polymer compound is 5 mg/g or more, the frequency of platelet adhesion can be significantly reduced as compared with a glass substrate or the like. When the amount of the intermediate water is 10 mg/g or more or 20 mg/g or more, the frequency of platelet adhesion can be remarkably reduced depending on the amount of the intermediate water. In particular, when the amount of the intermediate water is 30 mg/g or more, excellent prevention of platelet adhesion can be achieved.

When a coating composition containing a polymer compound according to the present invention is used to ensure adhesion to a base material, it is preferable that the polymer compounds according to the present invention are not bound to each other in a solvent by preparing a coating composition in which the polymer compound is dissolved in various solvents at a sufficiently dilute concentration. Further, it is preferable to promote adhesion of the silsesquioxane portion contained in the polymer compound according to the present invention to the surface of the substrate by evaporating and removing the solvent for a sufficient period of time after applying the coating composition to the substrate. It is also preferable to carry out chemical modification or the like for promoting adhesion of the silsesquioxane portion contained in the polymer compound according to the present invention to the surface of the substrate to be coated.

In the coating composition obtained by dissolving the polymer compound according to the present invention in various solvents, various substances can be mixed and used according to the purpose of coating within a range that does not significantly impair the biocompatibility or adhesion to the surface of the substrate. For example, in the polymer compound according to the present invention, by mixing and using the same polymer as the polymer chain bonded to the organic component (R) of silsesquioxane or a biocompatible polymer that is compatible with the polymer chain, it is possible to improve the biocompatibility while maintaining adhesion to the substrate surface.

It is also possible to use a coating composition containing a polymer compound according to the present invention as a so-called primer, and to laminate various polymers on the surface of the coating film.

The coating composition according to the present invention can coat the surface of various inorganic materials or the like to which biocompatibility is to be imparted with the polymer compound according to the present invention by evaporating and removing the solvent after coating the surface. When a biological substance such as blood is brought into contact with the surface coated with the polymer compound according to the present invention, it is preferable to hydrate the surface in advance so that the polymer portion containing a structure contributing to the inclusion of intermediate water is hydrated.

The coating composition according to the present invention may cover at least a part of the surface used in contact with living tissue or blood, and the coating composition according to the present invention can be used as a surface treatment agent for the surface of a base material constituting a medical device or the like.

It should be noted that in the present specification, a medical device is a device which is used for the purpose of not damaging the physiological activity indicated by the living body tissue or blood, etc., among devices which are used in contact with the living body tissue or blood, etc., and naturally includes, for example, a form in which the medical device is placed in a living body, a form in which the medical device is used in contact with the tissue or blood in a state in which the living body tissue is exposed, and a form in which the medical device is used in contact with blood which is an in-vivo component taken out from the body in an extracorporeal circulation medical material. In addition, the term "used for medical purposes" includes the above-mentioned "used in contact with living tissue or blood" or intended use thereof.

The material or shape of the member constituting the medical device or the like coated with the coating composition according to the present invention is not particularly limited, and may be, for example, a porous body, a fiber, a nonwoven fabric, a particle, a film, a sheet, a tube, a hollow fiber or a powder. Examples of the material include natural polymers such as wood brocade and hemp, nylon, polyester, polyacrylonitrile, polyolefin, halogenated polyolefin, polyurethane, polyamide, polycarbonate, polysulfone, polyethersulfone, synthetic polymers such as poly (meth) acrylate, ethylene-vinyl alcohol copolymer, butadiene-acrylonitrile copolymer, and mixtures thereof. In particular, the coating composition according to the present invention is preferably used for a surface such as a metal, a ceramic, a glass or a composite material thereof, which is difficult to ensure adhesion to a polymer material.

The coating composition according to the present invention can be used for medical devices which are used in contact with biological tissues or blood, and is preferably used for at least a part of surfaces in contact with biological tissues or blood, and preferably almost all surfaces in contact with blood, of medical devices such as implantable artificial organs, therapeutic devices, extracorporeal circulation artificial organs, and catheters (cardiovascular catheters such as angiography catheters, guide wires, and PTCA catheters, gastric tube catheters, gastrointestinal catheters, and esophageal tubes, digestive catheters such as tubes, urinary catheters, and urinary Suga catheters).

Further, an oxygen-containing gas exchange porous hollow fiber membrane may be housed in a housing, and the outer surface or outer surface layer of the hollow fiber membrane may be coated with the coating composition according to the present invention in a blood perfusion oxygen-containing hollow fiber membrane oxygenator in which blood flows on the outer surface side of the hollow fiber membrane and oxygen-containing gas flows inside the hollow fiber membrane.

Also disclosed is a dialyzer having a dialysate circuit comprising at least one dialysate container filled with dialysate and at least one drainage container for recovering the dialysate, and a fluid delivery means for delivering the dialysate from the dialysate container as a starting point or from the drainage container as an ending point, wherein at least a part of the surface in contact with the blood may be coated with the coating composition according to the present invention.

Further, the coating composition according to the present invention may be used for the purpose of forming various diagnostic chips by coating the surface of a base material or the surface of a particle in contact with an aqueous solution containing various proteins or cells by utilizing the selective adsorption property of a surface having a predetermined amount of intermediate water against proteins or cells.

In addition, the surface composed of the polymer compound according to the present invention can be preferably used as a support for cell culture capable of adhering and maintaining cells in a preferred form by applying the coating composition according to the present invention. That is, a cell culture support having a surface composed of a polymer compound according to the present invention is not particularly limited as long as it is a cell that lives by adhering to a substrate, and can be applied to the cell culture of epidermal cells, digestive tract epithelial cells such as vascular endothelial cells, oral endothelial cells, esophageal epithelial cells, gastric epithelial cells, intestinal epithelial cells, etc., respiratory epithelial cells such as nasal cavity mucosal epithelial cells, tracheal epithelial cells, alveolar epithelial cells, etc., exocrine gland cells such as sweat gland cells, sebaceous gland cells, apocrine gland cells, mammary cells, etc., endocrine gland cells such as salivary gland epithelial cells, lacrimal gland cells, pancreatic islet cells, adrenal medullary cells, adrenocortical cells, pinealocytes, pituitary cells, thyroid cells, etc., endocrine gland cells such as liver cells, renal epithelial cells, pancreatic cells, adrenal cells, etc., visceral parenchymal cells, taste bud cells, olfactory epithelial cells, hair cells, etc., sensory organ cells, nerve cells, and glial cells such as astrocytes, Schwann cells, etc., muscular cells such as cardiac muscle cells, skeletal muscle cells, smooth muscle cells, etc., fibroblasts, interstitial cells, connective tissue cells, chondrocytes, osteoblasts, etc., mesenchymal cells such as thymic epithelial cells, uterine epithelial cells, ovarian follicle cells, fallopian tube epithelial cells, seminiferous tubule epithelial cells, Leydig cells, etc.

Further, the cell culture support having a surface composed of the polymer compound according to the present invention can be used for culturing various types of stem cells such as stem cells having pluripotency such as embryonic stem cells (ES cells), induced pluripotent stem cells (iPS cells), embryonic tumor cells (EC cells), embryonic reproductive stem cells (EG cells), nuclear transfer ES cells and somatic cell derived ES cells, tissue stem cells such as hematopoietic stem cells, bone marrow-derived mesenchymal stem cells, adipose tissue-derived mesenchymal stem cells, other interstitial tissue derived stem cells, Muse cells and neural stem cells, tissue stem cells having pluripotency, and progenitor cells in various tissues such as liver, pancreas, adipose tissue, bone tissue and cartilage tissue. In the culture of stem cells using a cell culture support having a surface composed of a polymer compound according to the present invention, the promotion or suppression of differentiation occurs according to the characteristics of the cultured stem cells due to the fact that the cells can be maintained by adhering in a preferred form, and therefore the cell culture can be carried out in accordance with the purpose of the culture.

The polymer compound according to the present invention can be synthesized, for example, by mixing a molecule (monomer) in which a radical vinyl polymerizable group is provided at the end of a structure contributing to the inclusion of intermediate water, with a silsesquioxane molecule having a structure having a high radical reactivity in at least a part of the organic component (R) thereof, and causing radical polymerization.

In addition to the phospholipid polar group contained in the MPC polymer, examples of the structure contributing to the inclusion of the intermediate water include PEG and a chain ether structure or a cyclic ether structure which is a constitutional unit of PEG. Examples of the vinyl polymerizable group include a vinyl group, an acrylate group, a methacrylate group, a vinylidene group, an acrylamide group, a methacrylamide group, an acryloyl group and a methacryloyl group.

Specific examples of the molecule (monomer) having a structure contributing to the inclusion of the intermediate water include, in addition to 2-methacryloyloxyethyl phosphorylcholine (MPC), a molecule that produces a polymer represented by formula (1), (3) or (4) by polymerization. Further, molecules such as alkoxyalkyl (meth)acrylamide and polyvinyl pyrrolidone (PVP) which form a polymer capable of containing intermediate water by introducing a side chain into an acrylic skeleton via an amide group are exemplified. Further, a so-called macromonomer in which a vinyl group or the like is bonded to a terminal of PEG can also be preferably used.

As the structure having a high radical reactivity bonded to the organic component (R) of the cleaved silsesquioxane molecule, a group capable of forming a bond by radical vinyl polymerization is preferably used, and a halogeno group such as chlorine or bromine, a silyl enol ether group, a nitroxide group, a dithiocarbonate group, a trithiocarbonate group or a benzophenone ketyl group can be used, and an alkylene group having a thiol group at the tip is particularly preferred.

Further, a silsesquioxane into which an alkanoyl halide group, dithiocarbonate group, trithiocarbonate group or nitroxide group is introduced is mixed with a monomer having a structure contributing to the inclusion of the above-mentioned intermediate water and subjected to atom transfer radical polymerization, reversible addition-cleavage chain transfer polymerization or nitroxide mediated polymerization in this state to obtain a polymer compound in which a polymer chain and a silsesquioxane are bonded via a covalent bond such as an ester bond or an amide bond.

As a cleaved silsesquioxane molecule having a predetermined structure in an organic component (R), for example, a random-type silsesquioxane molecule can form a random-type silsesquioxane in which an organic component (R) is bonded by dissolving a monomer in which the organic component (R) is bonded to a trialkoxysilane such as trimethoxysilane or triethoxysilane in an aqueous solution or a water-containing organic solvent and maintaining the temperature at a predetermined temperature to cause dehydration condensation.

At this time, it is possible to adjust the molecular weight of the silsesquioxane to be synthesized by adjusting the molecular weight of the organic component (R) portion bonded to the monomer to be used or promoting dehydration condensation by heating under reduced pressure, and it is preferable to form a silsesquioxane structure having an appropriate structure according to the use of the coating composition using the polymer compound according to the present invention.

By dissolving the above-synthesized polymer compound according to the present invention in an appropriate solvent, a composition used for coating can be obtained. A solvent capable of dissolving a polymer compound according to the present invention is determined mainly by a structure of a polymer chain bonded as an organic component (R) of silsesquioxane. For example, in the case of a polymer compound to which a polymer chain containing PMEA is bonded, a coating composition containing the polymer compound at a high concentration can be obtained by using a mixed solvent of methanol and ethanol. Further, in the case of a polymer compound to which a polymer chain containing MPC or PEG is bonded, since the polymer compound exhibits primary water solubility, the coating can be performed by dissolving the polymer compound in water in which the base material to be coated is immersed, whereby the polymer compound is adsorbed and deposited on the surface of the base material.

Hereinafter, a method according to the present invention will be described in more detail using Examples. It should be noted that the following embodiment is one form of the present invention, and the present invention is not limited to this embodiment.

### Example 1

As an example of a cleaved silsesquioxane, a polymer compound in which PMEA, which is a polymer exhibiting biocompatibility, was bonded to an organic component (R) of a random silsesquioxane was synthesized by the method described below. In addition, by changing the ratio of the silsesquioxane and the MEA monomer used in the synthesis, the molecular weight of the PMEA bonded as the organic component (R) of the silsesquioxane was varied to change the properties of the synthesized polymer compound.

The synthesis of random silsesquioxane was carried out as follows. (3-mercaptopropyl) trimethoxysilane (26.6g, 0.135 mol, Tokyo Chemical Industry Co., Ltd.), ultrapure water (9.74g, 0.541 mol), and formic acid (0.130g, 2.71 mol, Tokyo Chemical Industry Co., Ltd.) were mixed, stirred at room temperature for 1 hour, heated to 70 °C, and held for 24 hours. This was followed by aging at 110 °C for 4 hours under a reduced pressure of about 50 mmHg to obtain a colorless viscous liquid product.

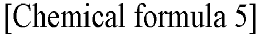

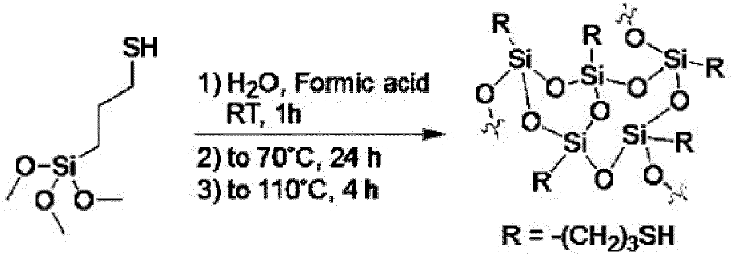

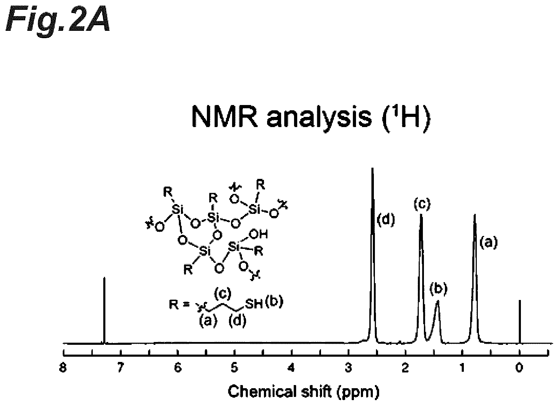

FIG. 1 shows the result of SEC analysis of the product obtained above. FIGS. 2A to C show the results of NMR analysis of the product obtained above. From the results of the SEC analysis, it can be seen that the average molecular weight of the molecules contained in the aqueous solutions was increased by 10 folds or more by the above-mentioned process, so that silsesquioxane containing about 15 silicon atom on average was formed.

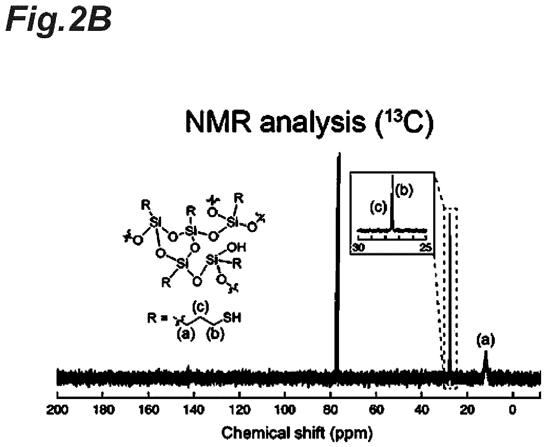

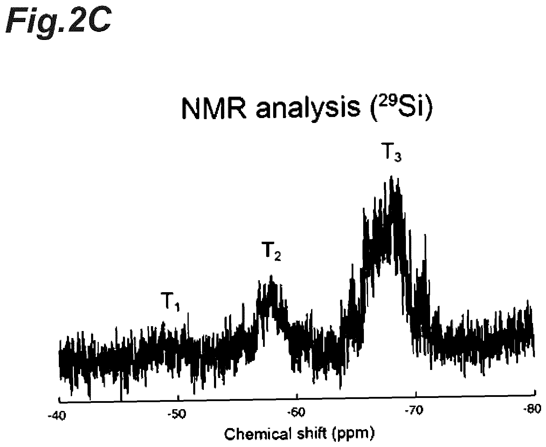

The NMR analysis shows a peak at the position corresponding to the Si-O bond (Fig. 2C), indicating that the increase in the average molecular weight is a result of hydrolytic polycondensation between trimethoxysilane molecules, and that silsesquioxane was synthesized. Since the peaks corresponding to T1 to T3 were broad, the synthesized silsesquioxane was considered to be of a random type. The half-width of the peak corresponding to T3 was 280 Hz. The NMR results show that (CH₂)₃SH bonded to trimethoxysilane is bonded as an organic constituent (R) of silsesquioxane (Fig. 2A, B).

Next, a polymer compound in which an MEA polymer was bonded to the organic component (R) was synthesized using the random silsesquioxane synthesized above in which the (CH₂)₃SH was bonded as the organic component (R). 2-Methoxyethyl acrylate (MEA) (15 g, 0.115 mol, Tokyo Chemical Co., Ltd.) was dissolved in 1,4-dioxane (Kanto Chemical Co., Ltd.) so as to have a monomer concentration of 2M with random silsesquioxane (SQ) having a thiol group synthesized above at the ratio shown in Table 1 and 2,2'-azoisobutyronitrile (ALBN) (0.95 mg, 5.78 µ mol, Tokyo Chemical Co., Ltd.) as a radical polymerization initiator. After removing dissolved oxygen by blowing argon gas for 30 minutes, the reaction was carried out at 60 °C for 6 hours.

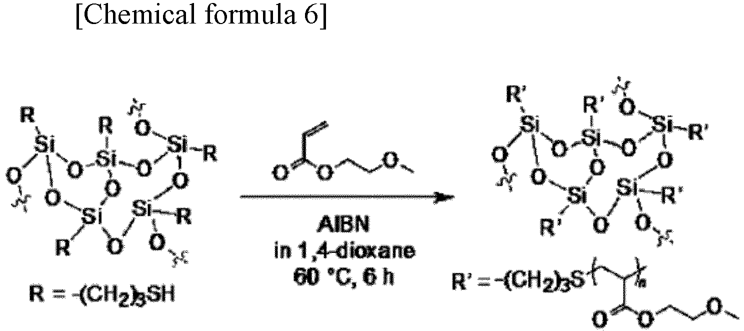

**[Table 1]**

| No. | MEA (g) | SQ (g) | AIBN (mg) | 1,4-dioxane (mL) | Theoretical value of Si content (%) |
|---|---|---|---|---|---|
| P1 | 15 | 0.087 | 0.95 | 42.7 | 0.5 |
| P2 | 15 | 0.174 | 0.95 | 42.5 | 1.0 |
| P3 | 15 | 0.528 | 0.95 | 42.2 | 3.0 |
| P4 | 15 | 0.870 | 0.95 | 41.8 | 5.0 |
| P5 | 15 | 1.74 | 0.95 | 41.0 | 10 |

After the above reaction solution was naturally cooled to room temperature, and poured into a large excess of mixed solvent of hexane (Kanto Chemical Co., Ltd.)/ethanol (Kanto Chemical Co., Ltd.) (volume ratio = 2:1) to precipitate the synthesized polymer compound. Reprecipitation was carried out several times using THF (Kanto Chemical Co., Ltd.) as a good solvent and a hexane/ethanol mixed solution (volume ratio = 2:1) as a poor solvent to remove residual reagents. Further, a large excess of ultrapure water was added and stirred for 12 hours to elute and remove the polymer of the low molecular weight fraction, and then dried under reduced pressure to obtain a desired polymer compound as a colorless viscous liquid.

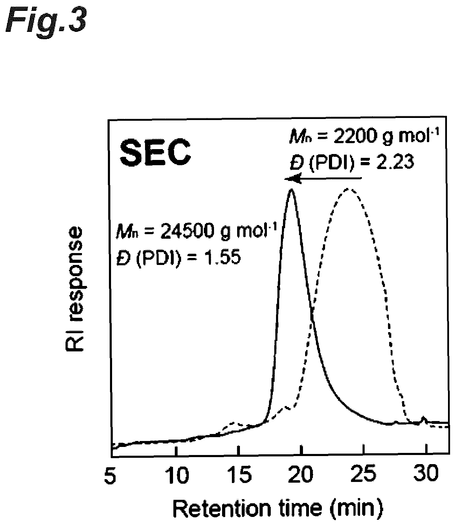

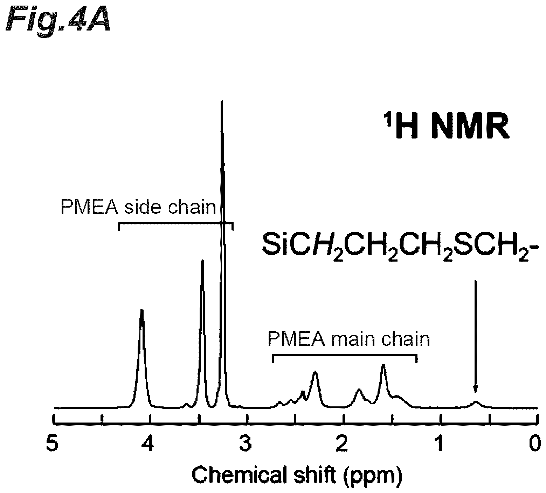

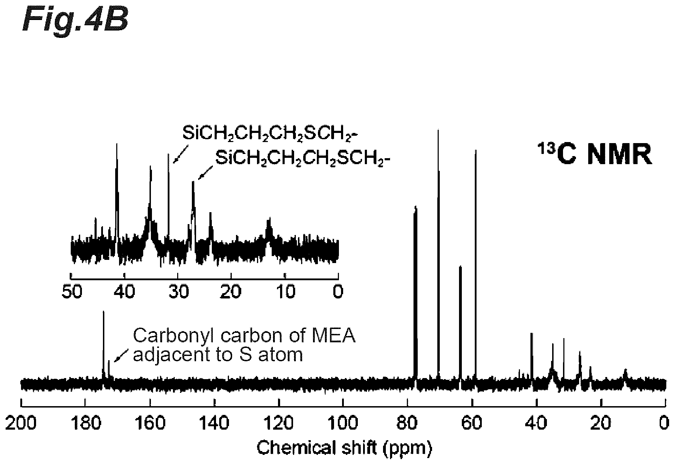

FIG. 3 shows the results of SEC analysis of a polymer compound synthesized under the conditions of P3 in Table 1 as an example of the polymer compound synthesized above. FIG. 4A and B show the results of NMR analysis of the polymer compound (P3). Table 2 shows the silicon atom content, the molecular weight of the polymer compound and the like for each polymer compound synthesized according to the preparation composition shown in Table 1.

The content of the silicon atom contained in the polymer compound described in Table 2 was determined by calculating the number of MEA units per silicon atom by comparing the number of protons of the side chains of the MEA polymer observed at around 3.2 to 4.2 ppm from the ¹H NMR measurement with the number of protons derived from silsesquioxane (2H) observed at around 0.75 ppm as the standard, and by using the formula (1/(number of PMEA units + 1) × 100 (%).

From the results of SEC analysis, it was found that the above process increased the molecular weight in comparison with the silsesquioxane used under all conditions, and this tendency was more remarkable under the condition where the amount of silsesquioxane used was small. The NMR analysis confirmed the presence of a carbonyl carbon adjacent to the S atom, indicating that the MEA polymer was bonded as an organic constituent (R) of the silsesquioxane by substituting the H atom of the thiol group.

**[Table 2]**

| No. | Si Content(a) (%) | Mw(b) (×10⁵g/mol) | Mn(b) (×10⁵g/mol) | PDI (Mw/Mn) |
|---|---|---|---|---|
| P1 | 0.8 | 11.37 | 6.81 | 1.67 |
| P2 | 1.5 | 8.73 | 5.39 | 1.62 |
| P3 | 5.0 | 3.80 | 2.45 | 1.55 |
| P4 | 10 | 2.63 | 1.73 | 1.52 |
| P5 | 25 | 0.90 | 0.66 | 1.36 |

| | | | | |
|---|---|---|---|---|
| a) Calculated from ¹H NMR b) Calculated from SEC measurement (PSt standard, THF, 40°) | | | | |

As shown in Table 2, as the amount of silsesquioxane (SQ) mixed in the polymerization of the MEA monomer increases, the molecular weight of the resulting polymer compound decreases. This is considered to indicate that the molecular weight of the MEA polymer portion bonded as the organic component (R) of each silsesquioxane decreases with an increase in the amount (number) of silsesquioxane which is the nucleus of polymerization.

Each of the polymer compounds produced above was saturated with water, and the transfer of latent heat in a temperature range of 50 to -100 °C was measured by a differential scanning calorimeter (DSC). The measurement was performed as follows. A predetermined amount (about 4 mg) of each polymer compound was spread thinly on the bottom of an aluminum oxide pan weighed in advance. As a sample, those saturated with water by immersing the polymer compound in water until the weight change due to water content did not occur were used. With regard to the aluminum oxide pans onto which each polymer was applied, the amount of generation and absorption of heat was measured with a differential scanning calorimeter (X-DSC7000, SII Inc.) by a process cooling the sample from 30C° to -100 °C, holding for 5 minutes, and then heating the sample from -100 °C to 50 °C at a heating rate of 5.0 °C/min.

For each sample, after the above measurement, the aluminum oxide pan was pin-holed and vacuum dried (VACUUM OVEN VOS-201SD, EYELA, TOKYO RIKAKIKAI Co.), and the weight after the vacuum drying was measured, By subtracting The weight of the aluminum oxide pan, the dry weight (g) of the polymer compound was obtained. On the other hand, in a result of the DSC measurement, the amount of heat generated due to the regularization (cold crystallization) of the intermediate water observed at around -30 to -60 °C was divided by the latent heat of solidification of water (Cp: 334 J/g) to obtain the amount (mg) of intermediate water contained in each polymer compound having saturated water content.

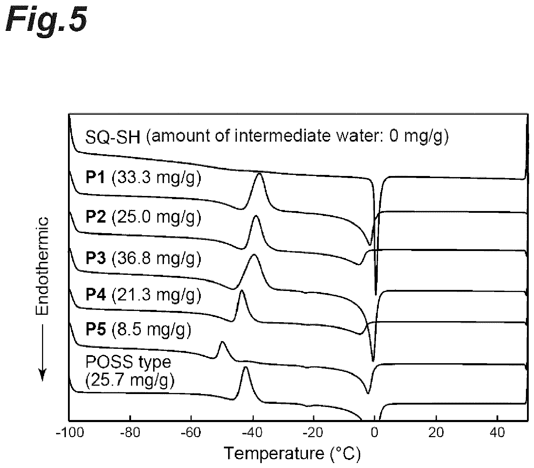

FIG. 5 shows a measurement result obtained by measuring each of the above-produced polymer compounds by DSC after the polymer compounds were saturated with water. FIG. 5 shows the amount of intermediate water per unit mass of each polymer compound obtained by dividing the heat quantity observed at around -30 to -60 °C by the latent heat of solidification of water in the measurement result of the DSC.

As shown in FIG. 5, in any of the polymer compounds PI to P5, a peak corresponding to the heat generated when the intermediate water is regularized below the freezing point is observed around -40 to - 50 °C, and it is confirmed that the intermediate water can be contained by the inclusion of water. On the other hand, in the silsesquioxane (SQ-SH in FIG. 5) to which the MEA polymer synthesized according to the scheme described in Chemical Formula 1 is not bonded, a peak corresponding heat generation below the freezing point is not observed, and it is shown that intermediate water is not contained when the silsesquioxane is hydrated.

The above results indicate that by bonding an MEA polymer as the organic component (R) of the random-type silsesquioxane, an intermediate water can be contained due to the MEA polymer portion.

An attempt was made to coat the glass surface using silsesquioxane in which the MEA polymer obtained above was bonded as an organic component (R) as follows. A silsesquioxane in which the MEA polymer obtained above was bonded as an organic component (R) was dissolved in a 2-propanol/water mixed solvent (volume ratio = 86.4:12.6) as a solvent at a concentration of 2.0 mg/mL to form a coating solution. The coating solution was dropped onto the surface of a glass substrate so as to be 32.5 µL/cm² and spin-coated (at 3000 rpm for 30 seconds).

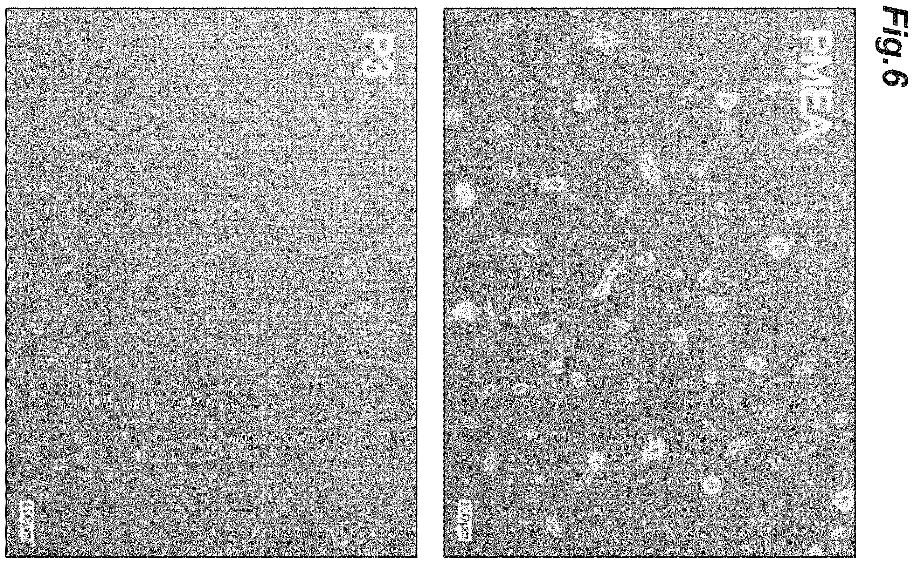

FIG. 6 shows the surface of the coated glass substrate. Although unevenness caused by peeling is observed on a glass substrate coated with PMEA, a uniform surface is observed on a glass substrate coated with the polymer compound (P3) according to the present invention, and it is understood that adhesion to the glass surface and the like is improved.

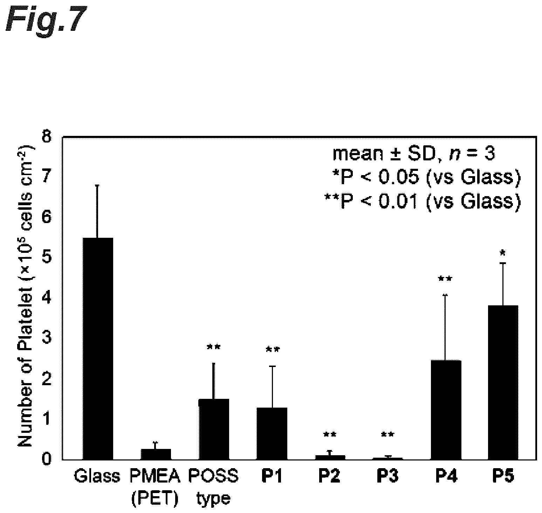

FIG. 7 shows the results of a platelet adhesion test on the surface of the coated glass substrate. The platelet adhesion test was performed in the following manner.

Human whole blood, an experimental blood purchased in the United States of America, was used for the experiment within 5 days after the blood was collected. Human whole blood that had been refrigerated was returned to room temperature by leaving it at room temperature for about 30 minutes. Thereafter, the boold was mixed by inversion three times, and centrifuged at 400 rcf for 5 minutes by a centrifuge (table top centrifuge 2420, KUBOTA). At this time, about 500 µ L of the supernatant (light yellow translucent) was collected and used as Platelet Rich Plasma (PRP). After sampling, the mixture was centrifuged at 2500 rcf for 10 minutes, and approximately 2 mL of the supernatant (light yellow clear) was sampled to use as Platelet Poor Plasma (PPP). The platelet concentration in PRP was calculated by counting the platelets in PRP diluted 800 times with PBS (-) using a hemacytometer, and PRP was diluted with PPP so that the seeding concentration was 3.0 × 10⁷ cells/cm² to prepare a platelet suspension. Platelets were adhered by placing 450 µL (about 300 µL/cm²) of the prepared platelet suspension on each substrate (sample) and incubating at 37 °C for 1 hour. The platelet suspension was then removed and washed twice with PBS. After that, the adherent platelets were immobilized on the substrate by immersion in 1% glutaraldehyde (25% glutaraldehyde, polyscience, Inc. 01909 diluted 1/25 with PBS (-)) solution and incubation at 37 °C for 2 hours. After immobilization, washing was carried out by immersing the cells one time each in PBS (-) (10 minutes), PBS (-) : water = 1 : 1 (8 minutes), and water (8 minutes, 10 minutes). After washing, the mixture was air-dried for 3 hours and then dried in a container containing silica gel for at least 1 day. After drying, platelet adhesion was counted by observing the substrate surfaces with a scanning electron microscope (SEM, KEYENCE, 3D real surface view microscope VE-9800).

As shown in FIG. 7, when an aqueous solution containing platelets or the like is brought into contact with a glass surface or the like which does not exhibit bioaffinity, remarkable platelet adhesion occurs. In contrast, it is known that on a water-containing PMEA surface, substantial platelet adhesion is not generated and biocompatibility is expressed. On the other hand, as shown in FIG. 6, in the case of PMEA coated on an untreated glass surface or the like, since peeling occurs after coating, it is difficult to coat the glass surface or the like with PMEA or the like to give bioaffinity.

In contrast, a polymer compound in which an MEA polymer is bonded to silsesquioxane having a silicon atom content of 1.5% and 5.0%, respectively, exhibits good adhesion to untreated glass surfaces and the like (FIG. 6), and the coated glass surfaces exhibit good biocompatibility with no substantial platelet adhesion observed in a platelet adhesion test.

On the other hand, a polymer compound in which an MEA polymer is bonded to silsesquioxane having a silicon atom content of 10% and 25%, respectively, exhibits good adhesion to untreated glass surfaces and the like, while the number of platelet adhesion in a platelet adhesion test increases with an increase in the silicon atom content.

The above results are considered to indicate that while adhesion to a glass surface or the like is developed by bonding an MEA polymer as an organic component (R) of a cleaved silsesquioxane, the content of intermediate water due to the MEA polymer decreases and the degree of expressed biocompatibility decreases with an increase in the ratio of the silsesquioxane.

From the above results, compared with the case of coating a polymer having biocompatibility such as PMEA, by using the polymer compound according to the present invention, the adhesion to a glass substrate or the like is improved and good coating can be achieved.

### Example 2

By the method described below, a silsesquioxane having a cage shape was synthesized (Chemical Formula 7), and a polymer compound in which an MEA polymer was bonded as its organic component (R) was formed in the same manner as in Example 1 (Chemical Formula 8).

Cage-type silsesquioxane was synthesized as follows. A mixture of (3-mercaptopropyl) trimethoxysilane (5.29g, 27 mmol) and concentrated hydrochloric acid (12M HCl aq) (10 mL, Kanto Chemical Co., Ltd.) dissolved in methanol (120 mL, Kanto Chemical Co., Ltd.) was heated at reflux for 24 hours. After that, the solvent was removed under reduced pressure, and the precipitated white viscous substance was washed well with methanol and then dissolved in 4 mL of THF. The solution was slowly added to 100 mL of acetonitrile (Kanto Chemical Co., Ltd.), and then allowed to stand for 30 minutes at - 80 °C and for 24 hours at - 20 °C, thereby obtaining a recystalized precipitate. It was collected and dried under reduced pressure to obtain a thiol-containing cage silsesquioxane as a white solid.

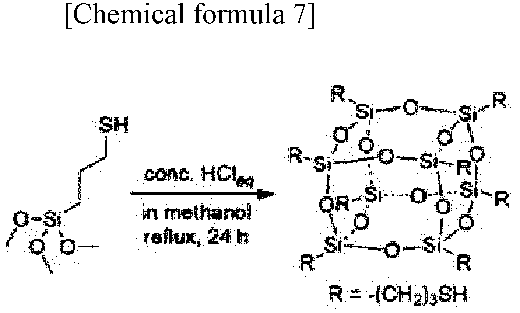

Next, MEA (1.5g, 11.5 mmol), thiol-containing cage silsesquioxane (0.053g, 0.052 mmol) synthesized above, and AIBN (0.095 mg, 0.578 µmol) were dissolved in 1,4-dioxane (Kanto Chemical Co., Ltd.) so that the monomer concentration was 2M. After removing dissolved oxygen by blowing argon gas for 30 minutes, the reaction was carried out at 60 °C for 6 hours.

After the above reaction solution was naturally cooled to room temperature, the solution was poured into a large excess of mixed solvent of hexane (Kanto Chemical Co., Ltd.)/ethanol (Kanto Chemical Co., Ltd.) (volume ratio = 2 : 1) to precipitate the synthesized polymer compound. Reprecipitation was carried out several times using THF (Kanto Chemical Co., Ltd.) as a good solvent and a hexane/ethanol mixed solution (volume ratio = 2 : 1) as a poor solvent to remove residual reagents. Further, a large excess of ultrapure water was added and stirred for 12 hours to extract and remove the polymer of the low molecular weight fraction, and then dried under reduced pressure to obtain a desired polymer as a colorless viscous liquid.

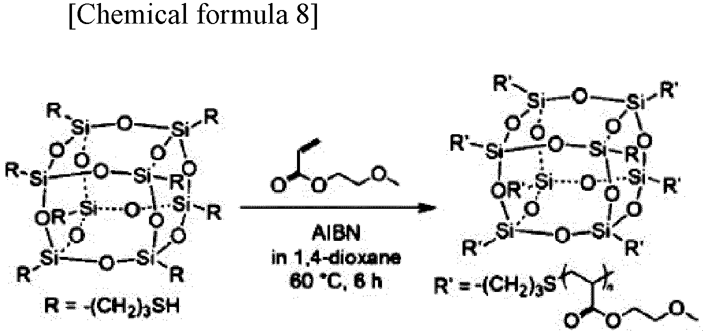

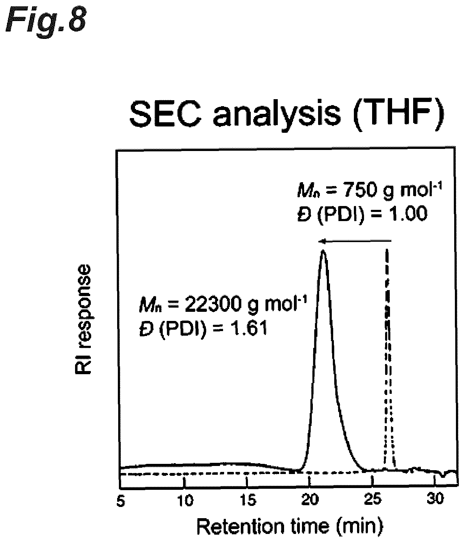

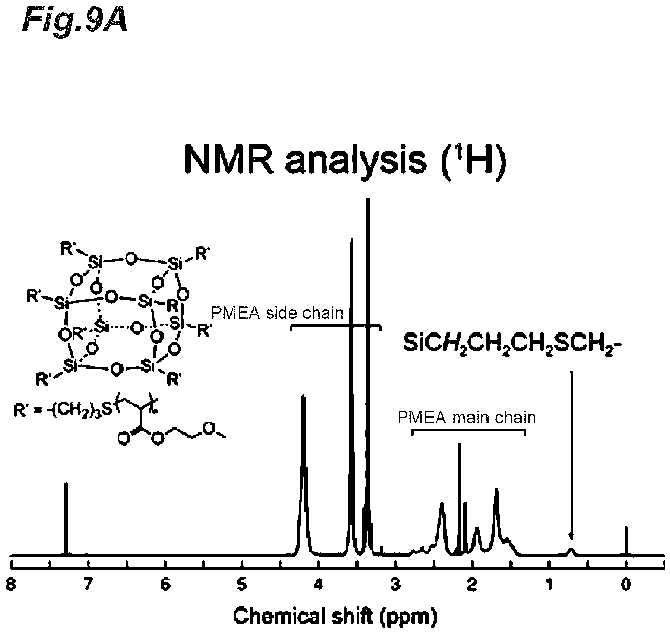

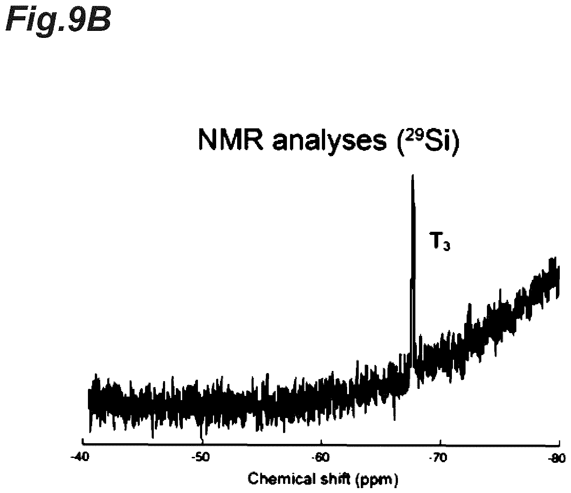

FIG. 8 shows the results of SEC analysis of the cage silsesquioxane synthesized above, and the cage silsesquioxane mixed with an MEA monomer and polymerized. Further, FIG. 9A and B show the results of NMR analysis of a polymer compound in which an MEA polymer is bonded to the organic components (R) of the above-mentioned cage silsesquioxane.

As shown in Fig. 9B, as a chemical shift corresponding to Si-O, only a T3 peak corresponding to a silicon atom in the structure in which all three silanols derived from trialkoxysilane are condensed to form (Si-O-Si) is observed sharply, and its full width at half maximum is 13.6 Hz. From this, it can be seen that the silsesquioxane synthesized above contains only a single type of Si-O bond and is a cage silsesquioxane.

In addition, from the fact that the molecular weight increases upon polymerization with the MEA monomer (FIG. 8) and the like, it was inferred that the MEA polymer is bonded as the organic component (R) of the cage silsesquioxane. The, the silicon atom content calculated in the same manner as in Example 1 was 5%.

The cage type silsesquioxane to which the MEA polymer synthesized above was bonded was dissolved in a solvent in the same manner as in Example 1, and the glass surface was coated.

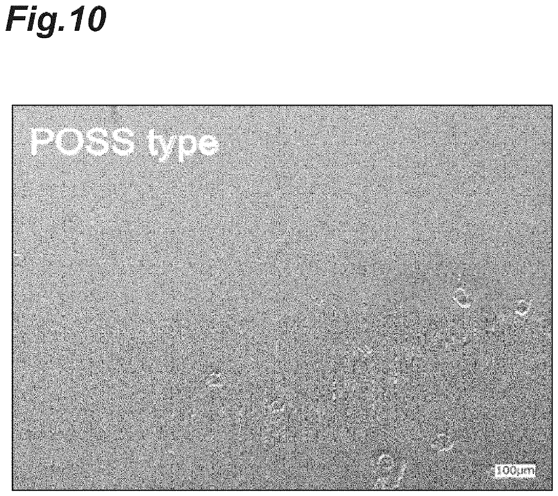

FIG. 10 shows a glass surface coated with a polymer compound in which an MEA polymer is bonded to an organic component (R) of a cage type silsesquioxane (POSS type). FIGS. 5 and 7 show a DSC measurement result of the cage silsesquioxane synthesized above measured in the same manner as in Example 1, a calculated intermediate water content, and a platelet adhesion test result in comparison with the case of using the cleavage type silsesquioxane.

From the above results, the existence of intermediate water was confirmed in a polymer compound in which an MEA polymer was bonded to an organic component (R) of a cage type silsesquioxane, and it was shown that biocompatibility can be imparted on a glass surface by coating.

### Comparative Example 1

A copolymer in which a chain ether structure contributing to the inclusion of intermediate water and a structure containing a trimethoxysilane portion contributing to the adhesion to the surface of an inorganic material coexisted in the side chain portion was synthesized by the method described below, and the adhesion to the glass surface and the expression of biocompatibility were evaluated.

MEA (15g, 0.115 mol), (3-acryloxypropyl) trimethoxysilane (1.35g, 5.76 mmol, Sigma-Aldrich), and AIBN (0.95 mg, 5.78 µmol) were dissolved in toluene (Kanto Chemical Co., Ltd.) so that the monomer concentration was 2M, dissolved oxygen was removed by blowing argon gas for 30 minutes, and then reacted at 60 °C for 5 hours. The reaction solution was cooled naturally to room temperature and then poured into a large excess of hexane to precipitate the polymer. The residual reagent was removed by reprecipitation several times using THF as a good solvent and hexane as a poor solvent. Further, a large excess of ultrapure water was added and stirred for 12 hours to extract and remove the polymer of the low molecular weight fraction, and then dried under reduced pressure to obtain a colorless viscous liquid product.

The product was considered to be a copolymer of MEA and (3-acryloxypropyl) trimethoxysilane.

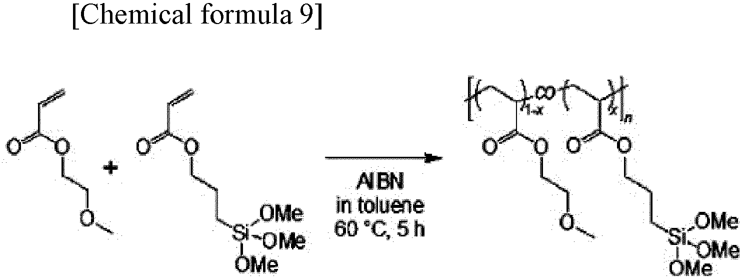

It was observed that the copolymer synthesized above was a viscous liquid immediately after synthesis and exhibited solubility in a solvent such as THF, while it rapidly hardened and lost solubility in the solvent.

In the copolymer synthesized above, it was inferred that partial crystallization or the like occurs by having a structure containing a portion having a siloxane bond exhibiting a strong polarity in a PMEA polymer containing a structure contributing to the inclusion of intermediate water.

### Industrial Applicability

The coating agent containing a polymer compound according to the present invention can easily impart bioaffinity to the surface of an inorganic material on the surface of a glass or the like.

## Claims

1. A polymer compound in which a polymer chain containing a structure contributing to the inclusion of intermediate water is bound as at least a part of an organic component R group of a silsesquioxane.

2. The polymer compound according to claim 1, wherein the silsesquioxane contains a structure in which a Si-O-Si bond is cleaved.

3. The polymer compound according to claim 1 or 2, wherein the polymer chain containing the structure contributing to the inclusion of the intermediate water is a polymer chain having at least one structure selected from a chain ether structure, a cyclic ether structure and a phospholipid polar group.

4. The polymer compound according to any one of claims 1 to 3, wherein the ratio of silicon atom contained in the silsesquioxane is 0.5% or more based on the sum of the number of the silicon atom and the number of units of a monomer constituting a polymer chain containing a structure contributing to the inclusion of the intermediate water.

5. The polymer compound according to any one of claims 1 to 4, wherein the mass of intermediate water contained when the polymer compound is saturated with water is 5 mg or more per unit mass of the polymer compound.

6. A coating composition comprising a polymer compound according to any one of claims 1 to 5 dissolved in a solvent.

7. A medical device wherein at least a part of the surface is coated with the polymer compound according to any one of Claims 1 to 5.

8. A cell culture support wherein at least a part of the surface is coated with the polymer compound according to any one of claims 1 to 5.

9. A method for synthesizing a polymer compound in which a polymer chain containing a structure contributing to the inclusion of intermediate water is bonded to at least a part of the organic component R group of a silsesquioxane, wherein radical polymerization is carried out by mixing a silsesquioxane having a functional group capable of radical vinyl polymerization with a monomer capable of radical vinyl polymerization that contains a structure contributing to the inclusion of intermediate water.
